# FASCICULE DE BREVET EUROPEEN

(11) **EP 1 827 403 B1**
(45) Date de publication et mention de la délivrance du brevet: **18.01.2012**
(21) Numéro de dépôt: 05825944.1
(22) Date de dépôt: 09.12.2005
(51) Int. Cl.: A61K 31/00, A61K 31/551, A61P 27/02

(54) **COMPOSITIONS POUR LE TRAITEMENT DES PATHOLOGIES OCULAIRES DE SURFACE**
ZUSAMMENSETZUNGEN ZUR BEHANDLUNG VON ERKRANKUNGEN DER AUGENOBERFLÄCHE
COMPOSITIONS FOR TREATING SURFACE OCULAR PATHOLOGIES

(30) Priorité: 09.12.2004 FR 0413136
(43) Date de publication de la demande: 05.09.2007
(73) Titulaire: Bueno, Lionel, 31840 Aussonne (FR); Droy-Lefaix, Marie-Thérèse, 60370 Hermes (FR); Caron, Philippe, 06140 Vence (FR)
(72) Inventeur: Bueno, Lionel, 31840 Aussonne (FR); Droy-Lefaix, Marie-Thérèse, 60370 Hermes (FR); Caron, Philippe, 06140 Vence (FR)
(74) Mandataire: Tezier Herman, Béatrice
(86) Numéro de dépôt international: PCT/FR2005/003095
(87) Numéro de publication internationale: WO 2006/061525

(56) Documents cités:
- EP-A- 1 417 976
- WO-A-97/30701
- WO-A-2005/112918
- US-A- 5 189 056
- US-A1- 2002 091 082
- DATABASE BIOSIS [Online] BIOSCIENCES INFORMATION SERVICE, PHILADELPHIA, PA, US; 2003, SATPATHY M ET AL: "THROMBIN - INDUCED INTER - ENDOTHELIAL GAP FORMATION IN BOVINE CORNEAL ENDOTHELIAL CELLS" XP002338320 Database accession no. PREV200300551552 & ARVO ANNUAL MEETING ABSTRACT SEARCH AND PROGRAM PLANNER, vol. 2003, 2003, page Abstract No. 3447, ANNUAL MEETING OF THE ASSOCIATION FOR RESEARCH IN VISION AND OPHTHALMOLOGY; FORT LAUDERDALE, FL, USA; MAY 04-08, 2003
- OIKAWA T ET AL: "POTENT INHIBITION OF ANGIOGENESIS BY WORTMANNIN, A FUNGAL METABOLITE" EUROPEAN JOURNAL OF PHARMACOLOGY, AMSTERDAM, NL, vol. 318, 1996, pages 93-96, XP002906145 ISSN: 0014-2999
- HONJO MEGUMI ET AL: "A myosin light chain kinase inhibitor, ML-9, lowers the intraocular pressure in rabbit eyes" EXPERIMENTAL EYE RESEARCH, vol. 75, no. 2, août 2002 (2002-08), pages 135-142, XP008050231 ISSN: 0014-4835
- WANG YUAN ET AL: "Activation of ERK1/2 MAP kinase pathway induces tight junction disruption in human corneal epithelial cells." EXPERIMENTAL EYE RESEARCH. JAN 2004, vol. 78, no. 1, janvier 2004 (2004-01), pages 125-136, XP008050258 ISSN: 0014-4835
- ROJANASAKUL Y ET AL: "THE CYTOSKELETON OF THE CORNEA AND ITS ROLE IN TIGHT JUNCTION PERMEABILITY" INTERNATIONAL JOURNAL OF PHARMACEUTICS (KIDLINGTON), vol. 68, no. 1-3, 1991, pages 135-150, XP008050244 ISSN: 0378-5173

## Description

La présente demande concerne des compositions pour le traitement de pathologies oculaires, en particulier des pathologies oculaires de surface chez les mammifères, notamment chez l'homme ou l'animal. L'invention concerne plus particulièrement des compositions permettant de réguler la perméabilité paracellulaire de l'épithélium oculaire de surface, correspondant au segment antérieur de l'oeil. Les compositions de l'invention reposent notamment sur l'utilisation d'agents ou de conditions modulant la tension du cytosquelette des cellules épithéliales de la conjonctive et de la cornée. L'invention est utilisable pour le traitement préventif ou curatif des affections oculaires de surface telles que les affections provoquées par une allergie (conjonctivite allergique par exemple) ou une inflammation, les conjonctivites infectieuses, les kératites variées et la maladie de l'oeil sec.

Au niveau du segment antérieur de l'oeil, l'épithélium de la cornée et celui de la conjonctive sont non kératinisés et de type stratifié. Ils protègent l'oeil des agresseurs extérieurs, la surface oculaire étant une muqueuse de transition entre le milieu oculaire profond et le milieu extérieur. Cet épithélium est une barrière anatomique et fonctionnelle qui, de par sa structure et la qualité de l'interface avec le film lacrymal, protège les constituants conjonctivaux, cornéens ainsi que le milieu endo-oculaire. Cet épithélium est une barrière compétitive entre la perte de liquide et l'entrée des pathogènes. Elle protège aussi l'oeil de toute abrasion. Pour que cette barrière soit efficace, les cellules constituant l'épithélium doivent adhérer étroitement les unes aux autres. Elles doivent également adhérer aux constituants cellulaires sous-jacents (Apostol S. et carstocea B. Oftalmologia 1994 ; 38(2) 101-6). Etant donné la position vulnérable qu'occupe l'épithélium situé à l'extérieur de l'oeil, la réponse de l'épithélium à toute agression doit être rapide et efficace.

Les inventeurs ont découvert que la résistance de la barrière épithéliale oculaire décrite ci-dessus est associée à la perméabilité de cet épithélium. L'épithélium oculaire est l'unique centre d'échanges entre le milieu extérieur de l'oeil (essentiellement constitué de larmes) et le milieu intérieur. Ces échanges peuvent s'effectuer soit à travers les cellules de l'épithélium, soit par des réseaux parallèles. Ainsi, le transport d'eau ou d'électrolytes, ou encore l'absorption de petites molécules (poids moléculaire généralement inférieur à 1000 Da environ) au niveau de la muqueuse oculaire s'effectue par voie transcellulaire, i.e., à travers les cellules épithéliales. Par contre, l'absorption de grosses molécules et le passage d'antigènes et/ou de toxines, se fait principalement par voie paracellulaire, au niveau des « jonctions serrées » qui sont disposés entre les cellules épithéliales [(Gobbels M et al. Fortschr. Ophtamol 1990; 87 (6): 646-8), (Noske W et al. Arch clin Exp Ophtalmol 1994; 232 (110): 608-13), (Sugrue SP et Zieske JD.Exp Eye Res 1997; 64: 11-20); (Hamalunem M et al. Ophtalmol Vis Sci 1997; 38 (36): 27-34)].

Les jonctions serrées (JS) épithéliales [ou *« tight junction* », (TJ)] sont des structures de liaison entre les cellules bordant les épithéliums muqueux. Elles assurent et contrôlent, au niveau de l'oeil, le transport trans-épithélial paracellulaire du film lacrymal ainsi que de macromolécules variées (allergènes, irritants, toxines, microorganismes, etc.) vers les tissus de l'oeil. Ces structures souples, connectées aux éléments du cytosquelette composés de filaments d'actine et de myosine (Turner JR et al. Am J Physiol 1997 ; 273 (4Pt) :C1378-85), sont formées par l'association de protéines transmembranaires (occludine, claudines) et de protéines cytoplasmiques [(protéines zonula occludens ZO-1, ZO-2, cingulines), (Sugue SP et Zieske JD. Exp Eye Res 1997 ; 64 :11-20), (Yi X et al. Ophtalmol Sci Res 2000; 41 (13): 4093-100)]. En conséquence, l'épithélium de la surface oculaire, de par sa structure anatomique et la qualité de son interface avec le film lacrymal dont l'action est de drainer et d'éliminer en permanence les microorganismes, les corps étrangers et les cellules épithéliales desquamées, assure une fonction de barrière nécessaire à la protection des constituants cellulaires sous-jacents et du milieu endo-oculaire. Un certain nombre d'agresseurs peuvent toutefois nuire à la stabilité de cette barrière oculaire, provoquant des altérations de la perméabilité trans-épithéliale liées à des modifications de la perméabilité paracellulaire. Une perméabilité accrue favorise la pénétration accrue de certains allergènes, pathogènes et molécules chimiques vers les cellules sous jacentes.

Le stress oxydatif, en libérant des radicaux libres oxygénés, occupe une place importante dans la genèse des pathologies affectant la surface oculaire. Les radicaux libres sont des espèces chimiques très réactives, toxiques, qui altèrent les membranes des cellules épithéliales. L'anion superoxyde libéré à partir de l'oxygène moléculaire réagit avec le peroxyde d'hydrogène pour former le radical hydroxyle. Ce dernier réagit à son tour avec les acides gras polyinsaturés des membranes provoquant ainsi la formation de peroxydes lipidiques, très agressifs, à l'origine de profondes désorganisations membranaires [Fridovitch I. Science 1978 ; 201 (4359) : 875-80]. Des études *in vitro* montrent qu'un déficit en vitamine A est à l'origine d'une altération de la perméabilité avec réduction de la perméabilité paracellulaire au mannitol ³H, kératinisation de l'épithélium conjonctival (Huang AJ et al. Invest Ophtalmol Vis Sci 31 (3) : 429-35 1991) et perte des cellules caliciformes.

Dans les cas de sécheresse oculaire, différents facteurs peuvent être à l'origine d'une altération de l'épithélium. Ces affections oculaires peuvent être provoquées par une exposition à des radiations (ultraviolets A et B, rayons X, chirurgie photoréfractive), des bactéries, des virus, des champignons, des allergènes, le port de lentilles (Mc Namara NA et al. Br J Ophtamol 1998 ; 82 (4): 376-81). Elles peuvent avoir une origine génétique comme dans le syndrome de Gougerot-Sjögren.
Des altérations de la perméabilité paracellulaire cornéenne ont aussi été mises en évidence. Elles sont liées à une déshydratation aiguë ou chronique de la surface oculaire (Lofebalo L et al Int J Immunopathol Pharmacol 1999 ;12 (3) :133-7), Kabuyonna I et Arakawa T J Ocul Pharmacol Ther 2003 ; 19 (3) : 281-9).

La perméabilité de l'épithélium oculaire de surface, comme le montre l'utilisation de la peroxydase du raifort (HRP), peut être altérée par la présence de conservateurs de collyres ou de substances antiseptiques telles que les ammoniums quaternaires. Le chlorure de benzalkonium (BAC ou BAK) entrant dans la totalité des collyres multidoses, comme les collyres anti-glaucomateux, même à de très faibles doses, provoque aussi la lyse des membranes des cellules de la surface oculaire (Tonjum AM. Acta Ophtalmol (Copenh) 1975 ; 53 ( 3) : 335-47) et des altérations de la perméabilité paracellulaire.

En outre, des altérations de la perméabilité, tant au niveau de la conjonctive que de la cornée, peuvent survenir suite à des traumatismes de la surface oculaire, pendant les phases de cicatrisation. Ainsi, la réalisation d'une biopsie, provoque une augmentation de la perméabilité paracellulaire, corrélée à l'état de l'épithélium (Huang AJ et al. Invest Ophtamol Vis Sci 1990 ; 32 (3) : 633-39).

En conséquence, l'altération des jonctions serrées épithéliales de la surface oculaire peut être à l'origine d'une sensibilisation, certains allergènes, pathogènes et /ou molécules chimiques étant capables de traverser cet épithélium pour interagir avec les cellules immunitaires de l'oeil. Les conditions dans lesquelles ce transfert est possible sont peu documentées *in vitro* et aucune démonstration n'a été jusqu'à ce jour apportée de l'implication de ces jonctions *in vivo* dans le développement d'une sensibilisation. Il est toutefois connu que la présence accrue de microorganismes, d'allergènes et/ou de molécules chimiques est à l'origine de phénomènes allergiques et inflammatoires, souvent accompagnés de douleurs pouvant entraîner une pathologie chronique.

La présente invention résulte de la mise en évidence *in vivo* du rôle des jonctions serrées de l'épithélium dans les pathologies oculaires, en particulier des pathologies affectant la surface oculaire. L'ouverture des jonctions serrées provoquée par une réaction allergique consécutive à l'instillation d'un agent de dégranulation des mastocytes (produit 48/80 : condensat de N-methyl-p-methoxyphenethylamine et de formaldehyde) ou d'une substance chimique irritante comme le chlorure de benzalkonium, modifie la perméabilité paracellulaire de l'épithélium oculaire. La présente invention propose pour la première fois, une approche thérapeutique des pathologies oculaires, en particulier des pathologies oculaires de surface, basée sur l'utilisation de composés ou conditions permettant de moduler la tension du cytosquelette desdites cellules épithéliales oculaires. Ainsi, ces composés ou conditions permettent de moduler la tension du cytosquelette des cellules épithéliales oculaires ou de réguler directement, de préférence diminuer, voire bloquer, l'ouverture des jonctions serrées de l'épithélium oculaire. Cette approche permet notamment de contrôler l'ouverture ou la fermeture des jonctions serrées de l'épithélium oculaire, sans nécessairement recourir à une synthèse protéique *de novo* et/ou à des dégradations protéiques et/ou structurales importantes de l'épithélium. L'invention permet de réguler la perméabilité de la surface oculaire de manière spécifique, fine et réactive et aussi d'agir sur le transfert d'allergènes, de pathogènes et/ou de molécules chimiques vers les cellules de l'immunité. Les compositions selon l'invention sont particulièrement bien adaptées à l'obtention d'un effet biologique rapide et contrôlable dans le temps (réversible).

La réaction d'infiltration de polynucléaires neutrophiles dans l'humeur aqueuse consécutive au dépôt sur la cornée d'un agent de dégranulation des mastocytes, le produit 48/80, est caractérisée par une infiltration de cellules polynucléaires neutrophiles décrite dans la littérature (Allansmith et al. Acta Ophtalmol.1989 ; 192 : 145-153S). Les inventeurs ont montré que l'infiltration est associée à l'augmentation de l'activité de la myélopéroxydase (MPO), enzyme libérée par les neutrophiles activés, et que cette augmentation pouvait être prévenue par le traitement préalable à l'aide d'un inhibiteur de la contraction du cytosquelette des cellules épithéliales, le ML-7, ayant pour effet d'inhiber l'action de la kinase catalysant la phosphorylation des chaînes légères de myosine. Cet effet correspond à une réduction de la tension du cytosquelette des cellules épithéliales cornéennes induite par l'inflammation et ayant pour conséquence de supprimer ladite inflammation liée à l'accumulation de neutrophiles dans l'humeur aqueuse.
Dans une deuxième série d'essais, les inventeurs ont également démontré que l'irritation de la cornée par l'instillation d'une solution de chlorure de benzalkonium déposée pendant 10 minutes avant rinçage, entraînait une augmentation de la MPO dans l'oeil. Celle-ci, très importante même après 6 heures, est également supprimée par un traitement préalable à l'aide de ML-7 administré par voie intrapéritonéale.
Dans ces essais, il apparaît donc que le ML-7 est capable de prévenir l'inflammation en s'opposant à la pénétration oculaire des agents d'agression (P48/80 et chlorure de benzalkonium).

Un premier objet de l'invention réside donc plus particulièrement dans l'utilisation d'un composé modulant la tension du cytosquelette de cellules épithéliales oculaires, en particulier des cellules épithéliales oculaires de surface, pour la préparation d'un médicament destiné au traitement préventif ou curatif de pathologies oculaires de surface chez l'homme ou l'animal.

Un autre objet de l'invention réside dans une composition pharmaceutique comprenant au moins un composé modulateur de la tension du cytosquelette (en particulier modulateur de l'ouverture des jonctions serrées) de cellules épithéliales oculaires de surface et un excipient acceptable sur le plan pharmaceutique, ladite composition étant formulée pour une administration par voie locale (via par exemple un collyre, un gel, etc., applicable directement sur l'oeil).

L'invention réside également dans le traitement préventif ou curatif des pathologies oculaires, en particulier des pathologies oculaires de surface, comprenant l'administration à un mammifère, notamment à un sujet humain ou animal, d'une quantité efficace d'un composé modulant la tension du cytosquelette de cellules épithéliales oculaires.

L'invention repose sur l'utilisation de composés modulant (de préférence inhibant) la tension et l'état de contraction du cytosquelette des cellules de l'épithélium oculaire, en particulier des cellules épithéliales oculaires du segment antérieur de l'oeil. Comme indiqué précédemment, cette approche permet de contrôler l'ouverture et la fermeture des jonctions serrées de l'épithélium oculaire sans nécessairement recourir à une synthèse protéique *de novo* et/ou à des dégradations protéiques et/ou structurales importantes de l'épithélium.

Les protéines composant les jonctions serrées sont associées au cytosquelette des cellules qu'elles relient. L'invention permet de moduler la tension du cytosquelette chez des sujets atteints de maladies ou désordres oculaires afin d'agir de manière non destructrice et transitoire sur la perméabilité de l'épithélium oculaire. Ainsi, la contraction du cytosquelette favorise l'ouverture des jonctions serrées, tandis qu'un relâchement du cytosquelette (ou qu'une inhibition de la contraction) favorise la fermeture desdites jonctions.

On utilise donc préférentiellement dans le cadre de l'invention des composés qui modulent la contraction du cytosquelette des cellules épithéliales oculaires. Selon la condition à traiter, des composés inhibant ou au contraire activant ou favorisant la contraction du cytosquelette de cellules épithéliales oculaires seront utilisés.

L'activité du composé sur la tension du cytosquelette peut être directe ou indirecte, c'est-à-dire dirigée sur les constituants mêmes du cytosquelette ou sur des régulateurs de sa tension. Les composés agissant de manière directe sur la tension du cytosquelette sont préférés. Les composés présentant une activité sélective sur la tension du cytosquelette, c'est-à-dire typiquement les composés qui n'affectent pas directement la structure des protéines constitutives des jonctions serrées, sont particulièrement préférés.

Un composé est considéré, au sens de l'invention, comme modulant la tension du cytosquelette lorsqu'il module l'ouverture des jonctions serrées. Un effet inhibiteur de la contraction ou de la tension des filaments d'actine et/ou de myosine ne doit pas nécessairement être complet ou total. Il suffit qu'il diminue la contraction ou la tension du cytosquelette suffisamment pour réduire l'ouverture des jonctions serrées. Cette réduction correspond à une diminution minimale d'environ 25 %, préférentiellement d'environ 30%, de façon encore plus préférée d'environ 50% de la perméabilité paracellulaire de l'épithélium oculaire.

Les composés pouvant être utilisés dans le cadre de la présente demande permettent de moduler la tension du cytosquelette. Il s'agit d'un agent (e.g, d'une molécule) ou d'une combinaison ou association d'agents, tels que défini dans le revendications.

Ces composés inhibent (ou modulent) la contraction ou la tension des chaînes légères de myosine et/ou d'actine, ou inhibent (ou modulent) la dégradation de l'actine.

De tels composés sont des inhibiteurs de la kinase des chaînes légères de myosine (MLCK).

Ce sont des inhibiteurs sélectifs de MLCKà savoir, le composé ML-7 {1-(5-iodonaphtalène-1-sulfonyl)-1H-hexahydro-1,4-diazepine} (Makishima M. et al. Feb Lett 1991 ; 287: 175), ou le composé ML-9 (Wilson DP et al. 2001). Ces composés sont utilisés dans le traitement du glaucame (WO 97/30701; Exp. Eye Res. 2002, 75 : 135-142).

D'autres cibles agissant sur la tension du cytosquelette sont notamment les protéines de liaison à la myosine, telles que, par exemple, la cinguline, ou les molécules de jonction, telles que la cadherine-E, la catenine-alpha ou les desmosomes. La modulation de l'activité ou de l'expression de ces protéines permet de réguler la tension du cytosquelette .

Selon un autre mode de réalisation, on peut utiliser des composés inhibiteurs de la synthèse de protéines ou autres molécules assurant la liaison entre les protéines du cytosquelette et les protéines des jonctions serrées. Parmi les protéines des jonctions serrées, on peut citer notamment les protéines occludines, claudines, ZO-1 et ZO-2. Un moyen de moduler l'ouverture ou la fermeture des jonctions serrées réside donc dans la régulation de la synthèse des protéines de liaison entre le cytosquelette et les protéines des jonctions serrées. En stimulant cette synthèse, il est attendu un renforcement des liaisons entre les jonctions serrées et le cytosquelette, conduisant à une plus faible perméabilité de l'épithélium.

D'autres composés utilisables sont par exemple des inhibiteurs de kinases activées par les mitogènes (MAPKK), notamment de la kinase MEK1 ou de la kinase P13, tels que les composés PD098,059 {2-( Amino-3-methoxyphenyl)-4H-1-benzopyran-4one} (Alessai et al. J Biol Chem 1995: 270: 27589) ou LY294002 {2-(4-Morpholinyl)-8-phenyl-1(4H)-benzopyran-4-one} (Vlahos et al. J Biol Chem, 1994 ; 269:5241).

D'autres molécules utilisables pour réguler, de manière indirecte, la tension du cytosquelette sont des facteurs de croissance, tel que le facteur de croissance endothélial (EGF) ou certaines cytokines susceptibles d'être libérées par les immunocytes telles que les interleukines-1, -4, -13, ou les facteurs tels que IGF-1 ou l'interféron gamma.

Une autre approche permettant de réguler de manière indirecte la tension du cytosquelette repose sur l'utilisation de peptide GLP2 (« glucagon-like peptide 2») ou de ses dérivés, qui peuvent permettre d'altérer la perméabilité de l'épithélium oculaire par un effet indirect sur la contraction du cytosquelette. De même, certaines molécules agissant sur des récepteurs situés au pôle apical des cellules épithéliales (ex : récepteurs aux protéinases ; PAR-2) peuvent agir indirectement sur le cytosquelette.

L'invention comprend l'utilisation d'agents tels que décrits dans les revendications agissant de manière directe sur la tension du cytosquelette, notamment de molécules inhibitrices de la contraction du cytosquelette, en particulier de molécules inhibitrices de la contraction ou de la tension des chaînes légères de myosine et/ou d'actine, ou inhibitrices de la dégradation de l'actine.

Comme indiqué ci-avant, les composés utilisés sont avantageusement des molécules, qui peuvent être sous forme isolée ou sous forme de combinaison d'extraits biologiques, etc. Ces molécules peuvent être synthétiques, semi-synthétiques ou biologiques, notamment d'origine animale, virale, végétale ou bactérienne.

La présente invention peut être utilisée pour le traitement ou la prise en charge de pathologies ou désordres oculaires, en particulier d'affections oculaires de surface.

L'invention concerne en particulier l'utilisation d'un composé tel que décrit précédemment modulant la tension du cytosquelette de cellules épithéliales oculaires pour la préparation d'un médicament destiné à contrôler (de préférence à réduire) la perméabilité paracellulaire de l'épithélium oculaire chez des sujets atteints d'affections oculaires, en particulier d'affections oculaires de surface.

Les utilisations selon l'invention, mentionnées précédemment, sont particulièrement efficaces dans le traitement d'une affection oculaire choisie parmi une kératite, une conjonctivite, le syndrome de l'oeil sec et toute autre altération de la perméabilité paracellulaire de l'épithélium oculaire. Des exemples de situations susceptibles de provoquer une telle altération sont par exemple le port par un patient de lentilles de contact ou encore les phases de cicatrisation chez un sujet souffrant d'une atteinte traumatique ou chirurgicale de l'épithélium oculaires.

En particulier, la présente invention est particulièrement adaptée au traitement préventif ou curatif d'une sensibilisation à un allergène. L'allergie du segment antérieur de l'oeil est une pathologie oculaire fréquente qui concerne 20% des populations nord américaine et européenne. Le nombre de patients affectés par ce type d'allergie est sans cesse en évolution en raison des facteurs environnementaux et/ou de l'augmentation de la durée de vie. Au niveau de la cornée de l'oeil, cette allergie est à l'origine de kératites allergiques. Au niveau de la conjonctive, l'allergie est responsable de conjonctivites qui se traduisent par un oeil rouge.
Les conjonctivites allergiques sont de plusieurs types : on observe les conjonctivites saisonnières, les conjonctivites des habitations, les conjonctivites provoquées par le port de lentilles de contact (giganta-papillaire) telles que mentionnées précédemment, les conjonctivites atopiques et les conjonctivites ayant pour origine l'utilisation de cosmétiques.

L'inflammation provoquée par les radicaux libres, souvent libérés sous l'influence de facteurs environnementaux (pollution, air conditionné, agents chimiques, etc.), atteint la cornée et la conjonctive. C'est le cas dans la conjonctivite vernale, forme très répandue chez les enfants et les jeunes adultes. Il existe aussi une kératoconjonctivite atopique qui survient chez des patients plus âgés qui souffrent d'éruptions cutanées. Cette forme de conjonctivite non saisonnière peut occasionner des lésions graves de la cornée et de la conjonctive si elle n'est pas traitée.

La présente invention peut être utilisée pour le traitement ou la prise en charge de toutes les pathologies oculaires de surface engendrées par les microorganismes, telles que les conjonctivites bactériennes (les kératites sont des complications fréquemment induites par ce type de conjonctivite) et les conjonctivites virales (adenovirus), très souvent bilatérales (associées également le plus souvent à une kératite), avec photophobie intense, douleurs et rougeurs.

La présente invention peut également être utilisée pour le traitement préventif ou curatif ou la prise en charge des sécheresses oculaires avec forte composante immuno-inflammatoire. Plus de 10 millions de personnes aux USA (représentant 15 % de la population âgée de plus de 65 ans) souffrent de cette agression de la surface oculaire.
Dans cette pathologie, il faut distinguer deux types de syndromes :
- les syndromes secs simples sont engendrés par les facteurs environnementaux (pollution, radiations, air conditionné, port de lentilles, écrans d'ordinateurs, etc.), aggravés par l'âge, la ménopause et certains traitements.
- les formes les plus graves sont souvent associées à des facteurs génétiques comme le syndrome de Gougerot-Sjôgren. Elles aboutissent à une maladie chronique, très invalidante pour les patients et pouvant entraîner dans les cas les plus graves une cécité.
L'inflammation chronique est toujours omniprésente dans l'oeil ; elle peut être primaire comme dans le syndrome de Gougerot-Sjögren, ou secondaire comme dans la kératite sèche.

La présente invention peut être utilisée pour prévenir ou traiter toute inflammation de la surface oculaire chez des sujets exposés à des conservateurs (substances antiseptiques), en particulier chez les sujets présentant des signes d'intolérance liés à une inflammation clinique ou infraclinique provoquée par une telle exposition. Les conservateurs les plus connus sur le marché sont les ammoniums quaternaires comme le chlorure de benzalkonium (BAC) qui entre dans la composition des collyres multidoses dont ceux enregistrés pour le traitement du glaucome. Ces conservateurs, en provoquant la libération de radicaux libres et une apoptose des cellules oculaires, atteignent l'épithélium cornéen et stimulent l'infiltration de la conjonctive par des cellules inflammatoires.

Un autre objet particulier de l'invention réside dans l'utilisation d'un composé tel que défini ci-avant, pour la préparation d'un médicament destiné à réduire, chez un patient, le passage d'une molécule connue pour son effet inflammatoire tel qu'un conservateur de collyre multidoses (préférentiellement utilisé dans le traitement du glaucome).

La présente invention est utilisable de manière préventive chez des sujets présentant des prédispositions ou une sensibilité aux désordres mentionnés ci-dessus, ou de manière curative lors d'événements pathologiques se manifestant sous la forme de crise ou de manière chronique. Les compositions selon l'invention permettent d'atténuer les symptômes des sujets, en particulier leur souffrance, et/ou la cause de ces désordres.

Un autre objet particulier de l'invention réside ainsi dans l'utilisation d'un composé tel que défini ci-avant, pour la préparation d'un médicament destiné à réduire la perméabilité paracellulaire de l'épithélium oculaire chez des sujets souffrant de maladies inflammatoires oculaires aiguës ou chroniques.

La présente invention est utilisable de manière préventive et/ou curative par exemple chez des sujet âgés pour lesquels la perméabilité paracellulaire se trouve augmentée (Nzekwe EU et Maurice DM. J Ocul Pharmacol 1994 ; 10 (3) : 521-3).

La présente invention démontre de façon surprenante que la suppression de l'augmentation de la perméabilité paracellulaire associée à l'ouverture des jonctions serrées empêche l'apparition des désordres oculaires, en particulier des désordres oculaires de surface.

Un objet particulier de l'invention réside dans l'utilisation d'un composé tel que défini ci-avant, pour la préparation d'un médicament destiné notamment à réduire la perméabilité paracellulaire et la sensibilisation aux allergènes, aux pathogènes et/ou aux molécules chimiques, chez des sujets atteints ou sensibles aux allergies oculaires.

L'invention concerne également des méthodes de prévention ou de traitement des conditions pathologiques mentionnées ci-dessus, comprenant l'administration à un sujet atteint d'une pathologie oculaire ou sensible à de telles pathologies, d'un composé ou traitement tel que défini ci-avant. De préférence, le composé ou traitement est administré dans une dose efficace pour réduire la perméabilité paracellulaire de l'épithélium oculaire de surface, et/ou réduire la sensibilité à la douleur et/ou réduire la migration transépithéliale d'allergènes vers les tissus cornéens ou conjonctivaux.

Le composé peut être administré par différentes voies et sous différentes formes. Ainsi, le composé peut se présenter sous une forme liquide ou solide, typiquement sous forme de collyre, de gel, de suppositoire, de solution injectable ou sous une forme buvable, etc. On préfère des composés formulés de manière à pouvoir être administrés localement (collyre ou gel par exemple) ou encore par voie orale (solutés buvables, comprimés, ampoules, etc.). Bien entendu, d'autres formes d'administration telles que des injections (intraveineuses, intrapéritonéales, intradermiques, sous-cutanées, intramusculaires, intra-artérielles, etc.) sont possibles.

Les composés tels que définis dans la présente invention peuvent être utilisés seuls, en combinaison et/ou en association avec au moins un autre agent actif, tel que, par exemple, d'autres substances utilisées dans le traitement des pathologies oculaires, en particulier des pathologies oculaires de surface. On peut citer par exemple les larmes artificielles, certains antioxydants, les différentes formes de cyclosporine, les agents antiseptiques, antibiotiques ou antiviraux, etc. Ces différents agents peuvent être utilisés en combinaison thérapeutique, administrés sous forme séparée, combinée, étalée dans le temps ou concomitante.

Un objet particulier de l'invention concerne ainsi l'utilisation d'un composé tel que décrit précédemment, en combinaison avec une composition antiseptique, antibiotique ou antivirale, pour la préparation d'un médicament destiné à réduire la perméabilité paracellulaire de l'épithélium oculaire chez un sujet atteint par une pathologie oculaire engendrée par le passage de microorganismes, telle qu'une kératite bactérienne et/ou virale.

Un autre objet de l'invention réside dans une composition pharmaceutique comprenant au moins un composé modulateur de la tension du cytosquelette (en particulier de l'ouverture des jonctions serrées) de cellules épithéliales oculaires, en particulier de cellules épithéliales oculaires de surface, et un excipient acceptable sur le plan pharmaceutique, ladite composition étant formulée pour une administration par voie locale (collyre ou gel, par exemple). De préférence, la composition se présente sous forme de collyre et/ou gel. Des excipients adaptés à la formulation sous forme de gel ou de collyre peuvent être choisis parmi l'eau ppi, l'hydroxyde de sodium, le glycérol, l'hypromellose, l'alcool polyvinilique, le sorbitol, le gluconate de potassium, l'eau distillée, le chlorure de sodium, le borate de sodium, l'acide borique, l'acide citrique, l'hydrogénophosphate de sodium, la methylhydroxypropylcellulose, le polysorbate 20, le métabisulfite de sodium, l'édétate de sodium, le parahydroxybenzoate de méthyle, le chlorure de benzalkonium, l'huile de vaseline et le chlorbutanol. De manière préférée, l'excipient est choisi parmi le glycérol, le chlorure de benzalkonium, l'hydroxyde de sodium et l'eau ppi.

La quantité de composé modulateur de la tension du cytosquelette (en particulier de l'ouverture des jonctions serrées) de cellules épithéliales oculaires dans la composition selon l'invention varie dans une large mesure et en particulier en fonction de la nature du composé choisi, de l'état du sujet à traiter, de la pathologie à traiter et/ou de l'effet désiré. Ainsi, l'homme du métier est à même d'établir la quantité efficace de composé modulateur de la tension du cytosquelette (en particulier de l'ouverture des jonctions serrées) de cellules épithéliales oculaires dans la composition et/ou pour le traitement selon l'invention.

D'autres aspects et avantages de la présente invention apparaîtront à la lecture des exemples qui suivent, qui doivent être considérés comme illustratifs .

### LEGENDES DES FIGURES

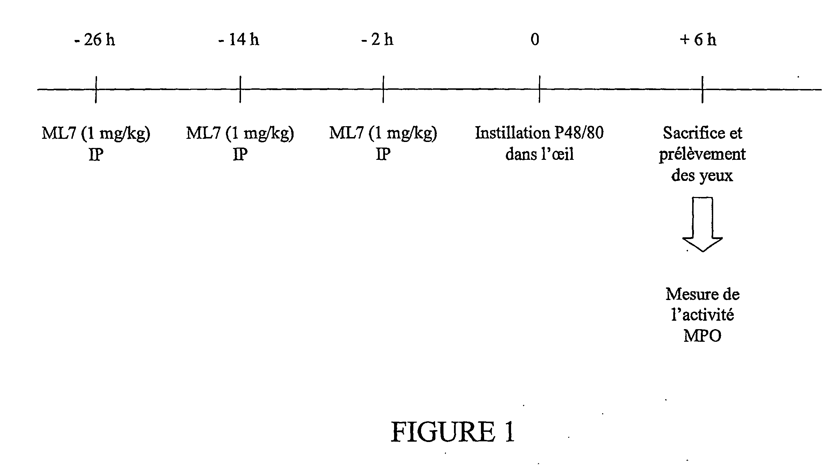

Figure 1 : Protocole expérimental de l'exemple 1. Les animaux ont reçu en Intra Péritonéale (IP), deux pré-traitements de ML7 (1 mg/kg) la veille de l'instillation du P48/80 (matin et soir) et un, 2 heures avant l'instillation.

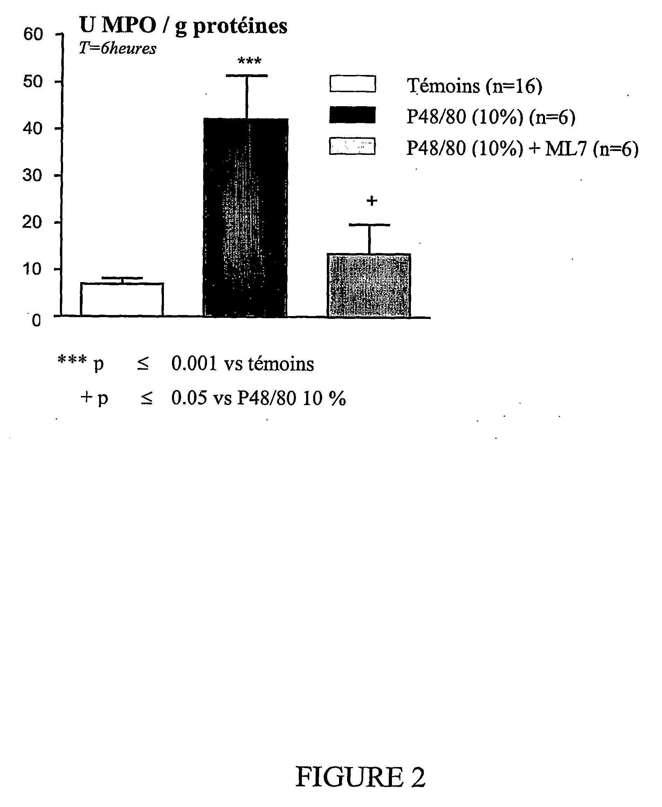

Figure 2 : Activité MPO (myélopéroxidase) de l'oeil et influence du P48/80 et du ML-7. Dans les conditions basales, l'activité MPO de l'oeil est faible (8,5 ±1,2 U/g.prot.) et les valeurs ne sont pas différentes entre les 2 yeux. Le traitement de l'oeil gauche par le P48/80 entraîne une forte augmentation de l'activité MPO (43,1 ±11,3 U/g.prot) correspondant à une augmentation de 407% traduisant l'accumulation de neutrophiles. Cette augmentation est supprimée par le ML-7.

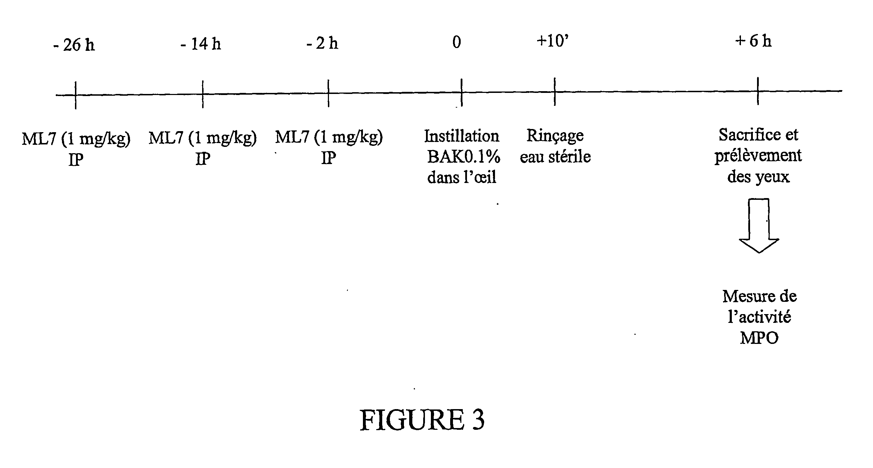

Figure 3 : Protocole expérimental de l'exemple 2. Les animaux ont reçu en IP, deux pré-traitements de ML7 (1 mg/kg) la veille de l'instillation du BAK 0.1% (matin et soir) et un, 2 heures avant l'instillation. Ils ont été sacrifiés 6 heures après le rinçage et les yeux ont été prélevés afin de mesurer l'activité MPO.

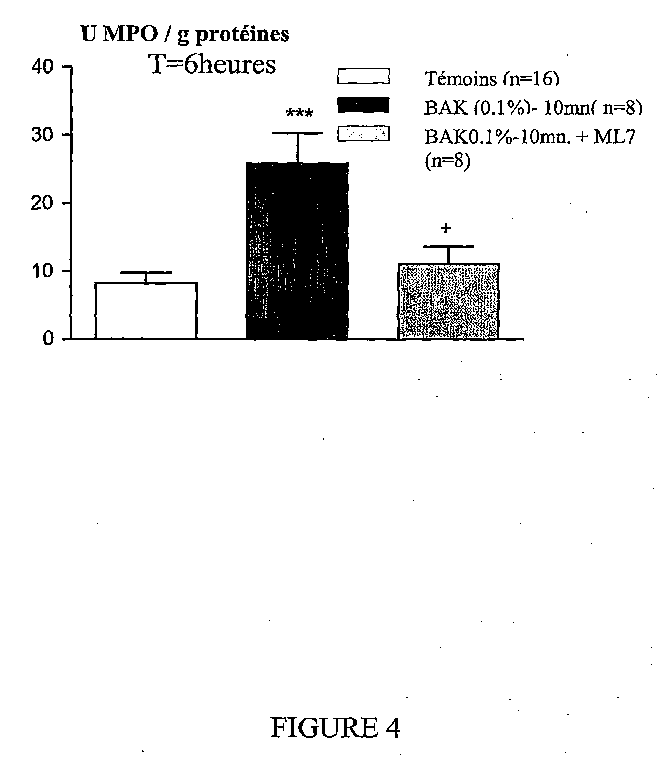

Figure 4 : Activité MPO (myélopéroxidase) de l'oeil et influence du chlorure de benzalkonium et du ML-7. En conditions basales, l'activité MPO de l'oeil est de 8.2 ± 1.9 U/ g. protéines. L'application dans l'oeil d'une solution à 0.1% de chlorure de benzalkonium entraîne, au bout de 6 heures, une augmentation très nette de la MPO totale de l'oeil (26.4 ± 7.2 U/g. protéines) correspondant à 321%. Cette augmentation est supprimée après traitement préalable par le ML-7 administré par voie IP.

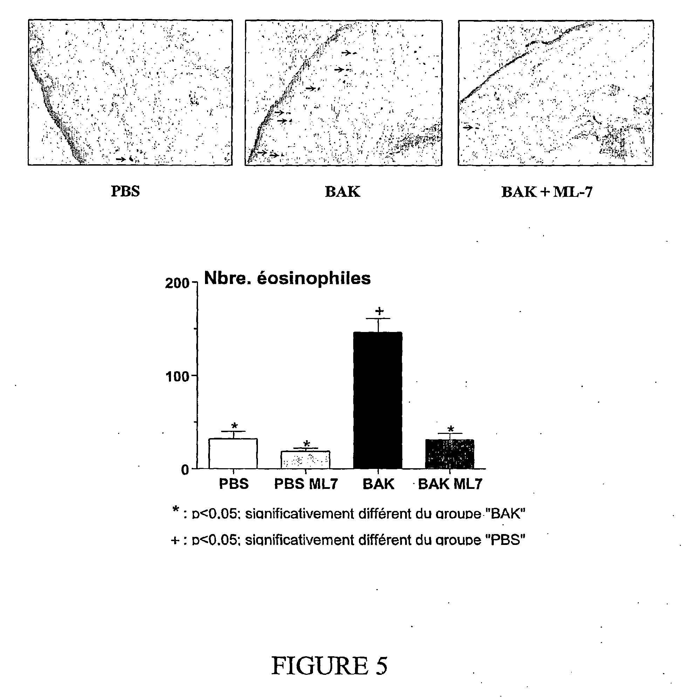

Figure 5 : Influence du ML-7 sur l'accumulation de polynucléaires éosinophiles à la jonction cornéo-conjonctive induite par le ML-7. Infiltration de polynucléaires éosinophiles induite par l'instillation de 10 µL de BAK. Au bout de 6 heures, l'augmentation d'éosinophiles est très significative. Cette infiltration d'éosinophiles est inhibée par le ML-7 administré localement. *En haut, images histologiques après coloration au Direct Red. En bas, comptage des éosinophiles au niveau du plexus veineux de la sc*/*ère, densité par mm2 (moyennes* ± *ESM ; n=8)*

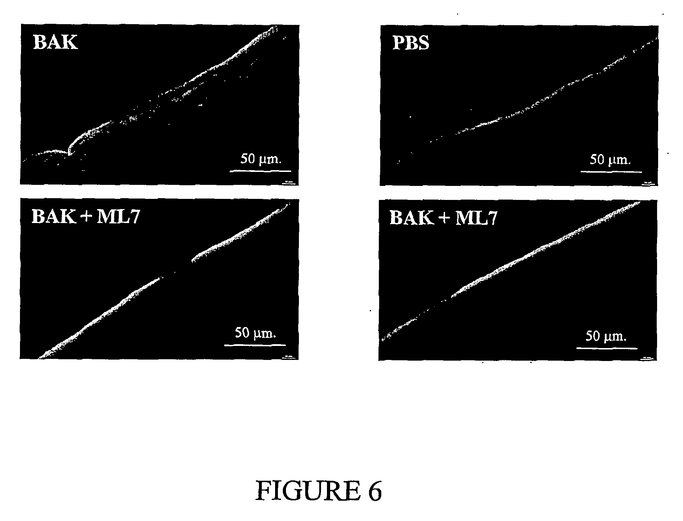

Figure 6 : Influence comparée du chlorure de benzalkonium (BAK), du PBS (solvant) et du BAK avec traitement préalable local avec le ML-7 sur le degré de pénétration du fluorochrome à travers la cornée chez le rat. Images obtenues par fluorescence de la surface externe de la cornée de rat observée après biotinylation *ex vivo* de l'oeil entier par l'avidine-fluorescéine après coupe en congélation (6 µm) (face externe de la cornée vers le haut)

### EXEMPLES

### Exemple 1 : Effets d'un inhibiteur de la MLCK (ML-7) administré par voie systémique (IP) sur l'inflammation oculaire induite par l'application sur la cornée d'un produit de dégranulation mastocytaire.

Le modèle d'irritation cornéenne utilisé a fait l'objet d'une validation chez le rat (Allansmith et al. Acta Ophtalmol, 1989 ; 192S : 145-153) et chez le lapin (Bucolo et al. J. Ocul. Pharmacol. 1993 ; 9 : 321-332).

### a) Matériel et méthodes :

*Animaux :* Trois lots de 8 rats mâles Wistar (200-250 g) placés dans des cages individuelles ont été utilisés. Les animaux ont reçu une alimentation standard (UAR, Villemoisson, Epinay sur Orge) et de l'eau à volonté.

*Induction de l'inflammation :* L'inflammation a été induite par instillation dans l'oeil de 10µl d'une solution de P48/80 à 10% (0,1g/ml). L'oeil témoin a reçu 10 µl de PBS 1X.

*Mesure de l'activifé myélopéroxidase :* Les yeux isolés ont été homogénéisés dans du tampon phosphate à l'aide d'un polytron. Ils ont ensuite subi trois cycles de congélation (azote liquide)/décongélation (bain-marie à 37°C). Après centrifugation (15mn, 10 000 tpm, 4°C), les culots ont été repris dans du tampon HTAB et soniqués pendant 10 secondes. Après une nouvelle centrifugation, l'activité MPO a été mesurée à partir des surnageants et d'un tampon réactionnel contenant de l'O-dianisidine hydrochloride et du peroxyde d'hydrogène à 0,0005%. Le changement d'absorbance à 460 nm a été mesuré à l'aide d'un spectrophotomètre

*Protocole expérimental :* Les animaux ont reçu en IP, deux pré-traitements de ML7 (1 mg/kg) la veille de l'instillation du P48/80 (matin et soir) et un, 2 heures avant l'instillation.

Ils ont été sacrifiés 6 heures après l'instillation et les yeux ont été prélevés afin de mesurer l'activité myélopéroxydase (MPO) totale de l'oeil entier (cf. Figure 1).

### b) Résultats (cf. : Figure 2) :

Dans les conditions basales, l'activité MPO de l'oeil est faible (8,5 ± 1,2 U/g.prot.) et les valeurs ne sont pas différentes entre les 2 yeux. Le traitement de l'oeil gauche par le P48/80 entraîne une forte augmentation de l'activité MPO (43,1 ± 11,3 U/g.prot) correspondant à une augmentation de 407% traduisant l'accumulation de neutrophiles. Cette augmentation est supprimée par le ML-7.

### Exemple 2: Effets d'un inhibiteur de MLCK (ML-7) sur l'inflammation oculaire (infiltration de neutrophiles) induite par l'instillation sur la cornée de chlorure de benzalkonium.

Le modèle d'irritation cornéenne par les sels de benzalkonium (BAK) a été largement utilisé tant *in vitro* qu'*in vivo.* Le protocole utilisé est issu d'une étude *in vitro* (Xu et al., 2000) adapté à l'étude *in vivo* chez le rat après essais préliminaires pour ajuster le temps et les concentrations.

### a) Matériel et méthodes :

*Animaux:* Des rats mâles Wistar (200-250 g) placés dans des cages individuelles ont été utilisés. Les animaux ont reçu une alimentation standard (UAR, Villemoisson, Epinay sur Orge) et de l'eau à volonté.

*Induction de l'inflammation :* L'inflammation a été induite par instillation dans l'oeil de 10µl d'une solution de BAK à 0,1% (1 mg/ml). L'oeil témoin a reçu 10 µl de PBS 1X. Dix minutes après l'instillation, les yeux ont été rincés avec 1 ml d'eau stérile.

*Mesure de l'activité myélopéroxidase :* Les yeux isolés ont été homogénéisés dans du tampon phosphate à l'aide d'un polytron. Ils ont ensuite subi trois cycles de congélation (azote liquide)/décongélation (bain-marie à 37°C). Après centrifugation (15mn, 10 000 tpm, 4°C), les culots ont été repris dans du tampon HTAB et soniqués pendant 10 secondes. Après une nouvelle centrifugation, l'activité MPO a été mesurée à partir des surnageants et d'un tampon réactionnel contenant de l'O-dianisidine hydrochloride et du peroxyde d'hydrogène à 0,0005%. Le changement d'absorbance à 460 nm a été mesuré à l'aide d'un spectrophotomètre

*Protocole expérimental :* Les animaux ont reçu en IP, deux pré-traitements de ML7 (1 mg/kg) la veille de l'instillation du BAK 0,1% (matin et soir) et un, 2 heures avant l'instillation. Ils ont été sacrifiés 6 heures après le rinçage et les yeux ont été prélevés afin de mesurer l'activité MPO (cf. Figure 3).

### b) Résultats (cf. Figure 4) :

En conditions basales, l'activité MPO de l'oeil est de 8,2 ±1,9 U/ g. protéines. L'application dans l'oeil d'une solution à 0,1% de chlorure de benzalkonium entraîne, au bout de 6 heures, une augmentation très nette de la MPO totale de l'oeil (26,4 ± 7,2 U/g. protéines) correspondant à 321%. Cette augmentation est supprimée après traitement préalable par le ML-7 administré par voie IP.

### Exemple 3 : Effets du ML-7 en application locale sur l'infiltration d'éosinophiles et la perméabilité des jonctions serrées, induites par le chlorure de benzalkonium (BAK), au niveau de l'oeil chez le rat.

### a) Matériel et méthodes :

*Animaux :* **Quatre** groupes de rats mâles Wistar (Janvier, Le Genest St Isle, France), pesant de 300 à 350 g ont été utilisés : BAK-carmellose sodique, BAK-ML-7, PBS-carmellose sodique, PBS-ML-7; le PBS et la carmellose sodique étant les solvants respectifs du benzalkonium (BAK) et du ML-7.

*Prétraitement au ML-7 :* Les animaux ont reçu une application locale de ML-7 (Sigma, France) 24 heures, 12 heures et 30 minutes avant induction chimique de l'inflammation oculaire. Ainsi, les animaux ont reçu sur chaque oeil 100 µg de ML-7 dans un volume de 10 µl de collyre (Carmellose sodique 4mg / 0,4mL) ou 10 µL de collyre seul.

*Induction de l'inflammation :* Trente minutes après la troisième application de ML-7 ou du collyre seul, les animaux ont reçu sur chaque oeil 10 µL de chlorure de benzalkonium (Sigma - Aldrich, Steinheim, Allemagne) à 0,5 % dans du PBS ou 10 µL de PBS. Après 10 minutes, les yeux de tous les rats ont été rincés avec 250 µL d'eau stérile.

*Prélèvement des yeux :* Six heures après l'application de chlorure de benzalkonium ou de PBS, les animaux ont été anesthésiés par administration intrapéritonéale de pentobarbital (80 mg/kg) (Ceva Santé Animale, Libourne, France), sacrifiés par décapitation et les yeux immédiatement prélevés pour congélation directe ou après biotinylation de surface (test de perméabilité des jonctions serrées).

*Mesure de l'infiltration de polynucléaires éosinophiles:* Les leucocytes polynucléaires éosinophiles ont été spécifiquement colorés au Direct Red et dénombrés dans la région du plexus veineux de la sclère. Ainsi, immédiatement après leur prélèvement, les yeux ont été inclus dans un milieu protecteur pour tissus en congélation (Tissu Tek ® OCT compound, Sakura Finetek, Inc., CA, USA), congelés dans l'azote liquide et conservés à -80°C. Des coupes de 6 µm d'épaisseur ont été ensuite réalisées au cryostat et fixées dans l'acétone froide pendant 10 minutes. Après séchage, les coupes ont été hydratées par des bains successifs dans le toluène (5, 3 et 2 minutes), puis l'éthanol à 100 % (3 et 2 minutes), l'éthanol à 95 % (3 et 2 minutes) et l'éthanol à 50 % pendant 2 minutes. Les coupes ont été ensuite colorées pendant 20 minutes dans une solution de rouge Sirius à 0,03 % dans l'éthanol à 50 % (Direct Red 75 dye content 30 %, Sigma-Aldrich, Steinheim, Allemagne), rincées dans l'eau courante pendant 5 minutes puis montées dans un milieu aqueux (glycérol / PBS 50/50 [vol/vol]). Le comptage des éosinophiles, colorés en rose vif sur fond clair, dans la région du plexus veineux de la sclère, s'est effectué par observation au microscope Nikon Eclipse 90 i équipé d'une caméra digitale. (Nikon DXM1200F). La surface de la zone de comptage a été déterminée grâce au logiciel d'analyse d'images Nikon Lucia version 4.8 et les comptages exprimés en nombre d'éosinophiles/mm². La comparaison des résultats obtenus pour les quatre groupes expérimentaux a été réalisée par analyse de variance une voie, suivie d'un test de comparaison multiple de Bonferroni et les différences ont été considérées significatives quand p < 0,05.

*Mesure de la perméabilité des jonctions serrées - Biotinylation de surface :* La perméabilité des jonctions serrées de la cornée a été testée par une technique de biotinylation des protéines de surface. Le réactif de biotinylation choisi est soluble dans l'eau et contient une fonction espaceur aminocaproyl, réduisant l'encombrement stérique lors du couplage avec l'avidine. Ainsi, immédiatement après leur prélèvement, les yeux ont été mis à incuber pendant 30 minutes à température ambiante et sous agitation douce, dans une solution de Biotinamidohexanecarboxylate de sodium et de 3-sulfo-N-hydroxysuccinimide à 1 mg/mL, dans du PBS (Sigma-Aldrich, Steinheim, Allemagne). Les yeux ont été ensuite rincés trois fois dans du PBS, inclus dans un milieu protecteur pour tissus en congélation (Tissue-Tek ® OCT compound, Sakura Finetek, Inc. , CA, USA), congelés dans l'azote liquide et conservés à -80°C. Des coupes de 6 µm d'épaisseur ont été ensuite réalisées au cryostat et fixées dans l'acétone froide pendant 10 minutes. Après séchage, les coupes ont été marquées pendant 30 minutes à l'obscurité, avec de l'avidine D-FITC (Vector Laboratories, Inc., Burlingame, CA, USA) diluée 250 fois dans du PBS-Tween contenant 1 % de BSA et rincés 3 fois 5 minutes dans du PBS-Tween, toujours à l'obscurité. Les lames ont été ensuite montées dans un milieu pour fluorescence (Cappel fluorostab embedding medium, MP Biomedicals, Inc., Aurora, Ohio, USA) et observées au microscope à fluorescence Nikon Eclipse 90 i équipé d'une caméra digitale (Nikon DXM1200F). Les images ont été traitées grâce au logiciel d'analyse d'images Nikon Lucia version 4.8. Aucune différence significative d'épaisseur de la cornée n'ayant été observée sur les coupes entre les différents groupes expérimentaux (102 ± 10 µm, 110 ± 9 µm, 115 ± 13 µm et 124 ± 8 µm pour les groupes BAK-carmellose sodique, BAK-ML-7, PBS-carmellose sodique et PBS-ML-7 respectivement), la profondeur du marquage fluorescent reflète la perméabilité des jonctions serrées de l'épithélium cornéen externe au réactif de biotinylation.

### b) Résultats : (cf. Figures 5 et 6)

### a) Mesure de l'infiltration de polynucléaires éosinophiles : (cf. Figure 5)

L'application dans l'oeil d'une solution de 10 µL de chlorure de benzalkonium entraîne après 6 heures, une augmentation très significative du nombre de leucocytes polynucléaires éosinophiles, colorés au Direct Red, dans la région du plexus veineux de la sclère, ce qui démontre une forte inflammation oculaire. Cette infiltration des leucocytes polynucléaires éosinophiles est significativement inhibée après traitement préalable par le ML-7 en application locale.

### c) Mesure de la perméabilité des jonctions serrées : (cf. Figure 6)

L'application dans l'oeil d'une solution de 10 µL de chlorure de benzalkonium favorise, après 6 heures, l'ouverture des jonctions serrées de l'épithélium cornéen qui se traduit par une pénétration plus profonde du marqueur fluorescent et de sa diffusion. Le traitement préalable par le ML-7 supprime cette augmentation de la diffusion et l'épaississement de la zone fluorescente.

## Revendications

1. Utilisation d'un inhibiteur sélectif de MLCK, pour la préparation d'un médicament destiné au traitement préventif ou curatif de pathologies oculaires de surface chez l'homme ou l'animal, ledit inhibiteur sélectif de MLCK étant le composé ML-7 ou ML-9.

2. Utilisation selon la revendication 1, pour la préparation d'un médicament destiné à contrôler la perméabilité paracellulaire de l'épithélium oculaire chez des sujets atteints d'affections oculaires de surface.

3. Utilisation selon la revendication 2, pour la préparation d'un médicament destiné à réduire la perméabilité paracellulaire de l'épithélium oculaire chez des sujets atteints d'affections oculaires de surface.

4. Utilisation selon la revendication 1 ou 2, **caractérisée en ce que** l'affection oculaire est choisie parmi une kératite, une conjonctivite et le syndrome de l'oeil sec.

5. Utilisation selon la revendication 3, pour la préparation d'un médicament destiné à réduire la perméabilité paracellulaire de l'épithélium oculaire chez un patient portant des lentilles de contact.

6. Utilisation selon la revendication 3, en combinaison avec au moins un agent antiseptique, antibiotique ou antiviral, pour la préparation d'un médicament destiné à réduire la perméabilité paracellulaire de l'épithélium oculaire chez un sujet atteint par une pathologie oculaire engendrée par le passage de microorganismes, telle qu'une kératite bactérienne et/ou virale, lesdits agents étant administrés sous forme séparée, combinée, étalée dans le temps ou concomitante.

7. Utilisation selon la revendication 3, pour la préparation d'un médicament destiné à réduire la perméabilité paracellulaire de l'épithélium oculaire chez des sujets exposés à des conservateurs.

8. Utilisation de la revendication 3, pour la préparation d'un médicament destiné à réduire la perméabilité paracellulaire de l'épithélium oculaire pendant les phases de cicatrisation, chez un sujet souffrant d'une atteinte traumatique ou chirurgicale de l'épithélium oculaire.

9. Utilisation selon l'une quelconque des revendications précédentes, **caractérisée en ce que** le composé est administré par voie orale, locale, intraveineuse ou intrapéritonéale.

## Claims

1. Use of a selective inhibitor of MLCK, for preparing a medicament for preventive or curative treatment of ocular surface pathologies in humans or animals, said selective inhibitor of MLCK being compound ML-7 or ML-9.

2. Use according to claim 1, for preparing a medicament for controlling paracellular permeability of the ocular epithelium in subjects with ocular surface disorders.

3. Use according to claim 2, for preparing a medicament for reducing paracellular permeability of the ocular epithelium in subjects with ocular surface disorders.

4. Use according to claim 1 or 2, wherein the ocular disorder is selected from keratitis, conjunctivitis and dry eye syndrome.

5. Use according to claim 3, for preparing a medicament for reducing paracellular permeability of the ocular epithelium in a patient wearing contact lenses.

6. Use according to claim 3, in combination with at least one antiseptic, antibiotic or antiviral agent, for preparing a medicament for reducing paracellular permeability of the ocular epithelium in a subject with an ocular pathology due to the penetration of microorganisms, such as bacterial and/or viral keratitis, said agents being administered separately, in combination, spread out over time or concomitantly.

7. Use according to claim 3, for preparing a medicament for reducing paracellular permeability of the ocular epithelium in subjects exposed to preservative agents.

8. Use according to claim 3, for preparing a medicament for reducing paracellular permeability of the ocular epithelium during the healing phases, in a subject with a trauma or surgical wound to the ocular epithelium.

9. Use according to any one of the preceding claims, wherein the compound is administered by the oral, local, intravenous or intraperitoneal route.

## Patentansprüche

1. Verwendung eines selektiven MLCK-Hemmers zur Herstellung eines Medikamentes für die präventive oder kurative Behandlung von oberflächlichen Augenkrankheiten beim Menschen oder Tier, wobei besagter selektiver MLCK-Hemmer die Verbindung ML-7 oder ML-9 ist.

2. Verwendung gemäß Anspruch 1 zur Herstellung eines Medikamentes für die Kontrolle der parazellulären Permeabilität des Augenepithels bei Personen, die an oberflächlichen Augenerkrankungen leiden.

3. Verwendung gemäß Anspruch 2 zur Herstellung eines Medikamentes für die Verminderung der parazellulären Permeabilität des Augenepithels bei Personen, die an oberflächlichen Augenerkrankungen leiden.

4. Verwendung gemäß Anspruch 1 oder 2, **dadurch gekennzeichnet, dass** die Augenerkrankung aus einer Keratitis, einer Konjunktivitis und dem Syndrom des trockenen Auges ausgewählt ist.

5. Verwendung gemäß Anspruch 3 zur Herstellung eines Medikamentes für die Verminderung der parazellulären Permeabilität des Augenepithels bei einem Patienten, der Kontaktlinsen trägt.

6. Verwendung gemäß Anspruch 3 in Kombination mit wenigstens einem antiseptischen, antibiotischen oder antiviralen Agens zur Herstellung eines Medikamentes für die Verminderung der parazellulären Permeabilität des Augenepithels bei einer Person, die an einer Augenkrankheit leidet, die durch die Passage von Mikroorganismen hervorgerufen wird, wie eine bakterielle und/oder virale Keratitis, wobei besagte Agenzien getrennt, kombiniert, zeitlich verteilt oder gleichzeitig verabreicht werden.

7. Verwendung gemäß Anspruch 3 zur Herstellung eines Medikamentes für die Verminderung der parazellulären Permeabilität des Augenepithels bei Personen, die Konservierungsmitteln ausgesetzt sind.

8. Verwendung gemäß Anspruch 3 zur Herstellung eines Medikamentes für die Verminderung der parazellulären Permeabilität des Augenepithels während der Heilungsphasen bei einer Person, die an einer traumatischen oder chirurgischen Verletzung des Augenepithels leidet.

9. Verwendung gemäß einem der vorhergehenden Ansprüche, **dadurch gekennzeichnet, dass** die Verbindung über den oralen, lokalen, intravenösen oder intraperitonealen Weg verabreicht wird.
